# EUROPEAN PATENT APPLICATION

(11) **EP 4 129 332 A1**
(43) Date of publication of application: **08.02.2023**
(21) Application number: 21774814.4
(22) Date of filing: 26.03.2021
(51) Int. Cl.: A61K 39/395, C07K 16/18

(54) **METHOD FOR DISPLAYING BISPECIFIC ANTIBODY ON SURFACE OF MAMMALIAN CELL AND VECTOR**

(30) Priority: 27.03.2020 CN 202010228279
(71) Applicant: Ddbio. Co, Ltd., (Shang Hai), Pudong Shanghai 201210 (CN)
(72) Inventor: ZHOU, Chen, Shanghai 201210 (CN)
(74) Representative: Weickmann & Weickmann PartmbB
(86) International application number: PCT/CN2021/083248
(87) International publication number: WO 2021/190631

(57) **Abstract**

Provided are a method for constructing a bispecific antigen binding polypeptide expression vector and the bispecific antigen binding polypeptide expression vector produced according to the method. The method comprises: performing treatment by using restriction endonuclease of a specific recognition enzyme cutting site to obtain seven nucleic acid fragments having specific cohesive ends, and directionally ligating the nucleic acid fragments. Further provided is a method for establishing a polypeptide display library by using the expression vector. The display library can be used for effectively screening antibodies or antibody fragments.

## Description

### Field of the Invention

The present application relates to the field of biomedicine, and specifically to a method for constructing a bispecific antigen-binding polypeptide expression vector.

### Background of the Invention

Currently, the conventional method for developing bispecific antibodies generally involves screening specific antibodies against two targets separately, then pairing each of one group of specific antibodies with each of the other group of antibodies, expressing and purifying, and then analyzing and testing them for physicochemical or biological activity, etc. The conventional method has the problems of many screening times, high cost, long cycle time, and serious quality degradation after long-term preservation, making it difficult to meet the needs of industrial mass production.

Therefore, there is an urgent need for methods to screen bispecific antibodies that can meet the needs of industrial mass production, with controlled quality and simple operation.

### Summary of the Invention

The present application provides a method for displaying bispecific antigen-binding polypeptides (e.g., bispecific antibodies) on the surface of cells (e.g., mammalian cells). First, a bacterial library capable of expressing different components (e.g., antigen-binding polypeptides or fragments thereof against two or more different targets, expression vector components) is constructed, and the desired components are cleaved by using restriction endonucleases at specific restriction sites to obtain the corresponding vector fragments, which can be ligated directionally to form the bispecific antigen-binding polypeptide expression vector. The expression vector is transferred into a cell, so that the cell can display the bispecific antigen-binding polypeptide, and the expression of the bispecific antigen-binding polypeptide and its binding affinity to each antigen can be directly analyzed. By using the method of the present application, bispecific antigen-binding proteins can be successfully expressed on the surface of mammalian cells, and multiple peptide chains can be expressed simultaneously. The vector can be applied to the expression and screening of bispecific antigen-binding proteins of any structural forms, thereby promoting the screening and development of bispecific antibody drugs and improving the drug success rate.

In one aspect, the present application provides a method for constructing a bispecific antigen- binding polypeptide expression vector, which includes: a) providing a first polynucleotide comprising S5-LC1-S6 in the direction from 5' to 3'; b) providing a second polynucleotide comprising B4-VH1-B3 in the direction from 5' to 3'; c) providing a third polynucleotide comprising B2-LC2-B4 in the direction from 5' to 3'; d) providing a fourth polynucleotide comprising B5-VH2-B6 in the direction from 5' to 3'; e) providing a fifth polynucleotide comprising S6-expression vector fragment I-B2 in the direction from 5' to 3'; f) providing a sixth polynucleotide comprising B3-expression vector fragment II-B5 in the direction from 5' to 3'; g) providing a seventh polynucleotide comprising B6-expression vector fragment III-S5 in the direction from 5' to 3'; h) specifically cleaving the first polynucleotide, the second polynucleotide, the third polynucleotide, the fourth polynucleotide, the fifth polynucleotide, the sixth polynucleotide and the seventh polynucleotide with a restriction endonuclease to obtain a cleaved first polynucleotide, a cleaved second polynucleotide, a cleaved third polynucleotide, a cleaved fourth polynucleotide, a cleaved fifth polynucleotide, a cleaved sixth polynucleotide and a cleaved seventh polynucleotide; wherein the restriction endonuclease specifically recognizes S5, S6, B4, B3, B2, B5 and B6, respectively; i) mixing the cleaved first polynucleotide, the cleaved second polynucleotide, the cleaved third polynucleotide, the cleaved fourth polynucleotide, the cleaved fifth polynucleotide, the cleaved sixth polynucleotide and the cleaved seventh polynucleotide so that they can be ligated directionally and cyclized to form the expression vector; wherein the LC1 encodes a first light chain of the bispecific antigen-binding polypeptide, the VH1 encodes a first heavy chain variable region of the bispecific antigen-binding polypeptide, and the first light chain can bind to the first heavy chain variable region to form a first Fab for recognizing a first target; the LC2 encodes a second light chain of the bispecific antigen-binding polypeptide, the VH2 encodes a second heavy chain variable region of the bispecific antigen-binding polypeptide, and the second light chain can bind to the second heavy chain variable region to form a second Fab for recognizing a second target; wherein the B2, B3, B4, B5, B6, S5 and S6 are each independently recognition sites for the restriction endonuclease.

In some embodiments, the end produced from the specific cleavage of B2 by the restriction endonuclease that specifically recognizes it does not recognize or link to each other with the end produced from the specific cleavage of any one of the B3, B4, B5, B6, S5 and S6 by the corresponding restriction endonuclease.

In some embodiments, the end produced from the specific cleavage of B3 by the restriction endonuclease that specifically recognizes it does not recognize or link to each other with the end produced from the specific cleavage of any one of the B2, B4, B5, B6, S5 and S6 by the corresponding restriction endonuclease.

In some embodiments, the end produced from the specific cleavage of B4 by the restriction endonuclease that specifically recognizes it does not recognize or link to each other with the end produced from the specific cleavage of any one of the B2, B3, B5, B6, S5 and S6 by the corresponding restriction endonuclease.

In some embodiments, the end produced from the specific cleavage of B5 by the restriction endonuclease that specifically recognizes it does not recognize or link to each other with the end produced from the specific cleavage of any one of the B2, B4, B3, B6, S5 and S6 by the corresponding restriction endonuclease.

In some embodiments, the end produced from the specific cleavage of B6 by the restriction endonuclease that specifically recognizes it does not recognize or link to each other with the end produced from the specific cleavage of any one of the B2, B4, B5, B3, S5 and S6 by the corresponding restriction endonuclease.

In some embodiments, the end produced from the specific cleavage of S5 by the restriction endonuclease that specifically recognizes it does not recognize or link to each other with the end produced from the specific cleavage of any one of the B2, B4, B5, B6, BB3 and S6 by the corresponding restriction endonuclease.

In some embodiments, the end produced from the specific cleavage of S6 by the restriction endonuclease that specifically recognizes it does not recognize or link to each other with the end produced from the specific cleavage of any one of the B2, B4, B5, B6, S5 and B3 by the corresponding restriction endonuclease.

In some embodiments, the restriction endonuclease is selected from SfiI and BsmBI.

In some embodiments, the B2, B3, B4, B5 and B6 can be specifically recognized and cleaved by BsmBI.

In some embodiments, the S5 and S6 can be specifically recognized and cleaved by Sfil.

In some embodiments, the B2 includes a nucleic acid sequence as set forth in SEQ ID NO: 1.

In some embodiments, the B3 includes a nucleic acid sequence as set forth in SEQ ID NO: 2.

In some embodiments, the B4 includes a nucleic acid sequence as set forth in SEQ ID NO: 3.

In some embodiments, the B5 includes a nucleic acid sequence as set forth in SEQ ID NO: 4.

In some embodiments, the B6 includes a nucleic acid sequence as set forth in SEQ ID NO: 5.

In some embodiments, the S5 includes a nucleic acid sequence as set forth in SEQ ID NO: 6.

In some embodiments, the S6 includes a nucleic acid sequence as set forth in SEQ ID NO: 7.

In some embodiments, the method further includes introducing the first polynucleotide into a first bacterium to obtain an LC1 light chain component bacterial library. In some embodiments, the method includes inserting the first polynucleotide into a component vector to form an LC1 storage ligation product, and introducing the LC1 storage ligation product into the first bacterium to obtain the LC1 light chain component bacterial library. In some embodiments, the method further includes acquiring a first light chain component plasmid comprising the first polynucleotide from the LC1 light chain component bacterial library. In some embodiments, the method further includes acquiring the cleaved first polynucleotide from the first light chain component plasmid. In some embodiments, the method includes digesting the first light chain component plasmid with a restriction endonuclease that specifically recognizes the S5 and S6, thus obtaining the cleaved first polynucleotide.

In some embodiments, the method further includes introducing the second polynucleotide into a second bacterium to obtain a VH1 heavy chain component bacterial library. In some embodiments, the method includes inserting the second polynucleotide into a component vector to form a VH1 storage ligation product, and introducing the VH1 storage ligation product into the second bacterium to obtain the VH1 heavy chain component bacterial library. In some embodiments, the method further includes acquiring a first heavy chain component plasmid comprising the second polynucleotide from the VH1 heavy chain component bacterial library. In some embodiments, the method further includes acquiring the cleaved second polynucleotide from the first heavy chain component plasmid. In some embodiments, the method further includes digesting the first heavy chain component plasmid with a restriction endonuclease that specifically recognizes the B4 and B3, thus obtaining the cleaved second polynucleotide.

In some embodiments, the method further includes introducing the third polynucleotide into a third bacterium to obtain an LC2 light chain component bacterial library. In some embodiments, the method includes inserting the third polynucleotide into a component vector to form an LC2 storage ligation product, and introducing the LC2 storage ligation product into the third bacterium to obtain the LC2 light chain component bacterial library. In some embodiments, the method further includes acquiring a second light chain component plasmid comprising the third polynucleotide from the LC2 light chain component bacterial library. In some embodiments, the method further includes acquiring the cleaved third polynucleotide from the second light chain component plasmid. In some embodiments, the method includes digesting the second light chain component plasmid with a restriction endonuclease that specifically recognizes the B2 and B4, thus obtaining the cleaved third polynucleotide.

In some embodiments, the method further includes introducing the fourth polynucleotide into a fourth bacterium to obtain a VH2 heavy chain component bacterial library. In some embodiments, the method includes inserting the fourth polynucleotide into a component vector to form a VH2 storage ligation product, and introducing the VH2 storage ligation product into the fourth bacterium to obtain the VH2 heavy chain component bacterial library. In some embodiments, the method further includes acquiring a second heavy chain component plasmid comprising the fourth polynucleotide from the VH2 heavy chain component bacterial library. In some embodiments, the method further includes acquiring the cleaved fourth polynucleotide from the second heavy chain component plasmid. In some embodiments, the method includes digesting the second heavy chain component plasmid with a restriction endonuclease that specifically recognizes the B5 and B6, thus obtaining the cleaved fourth polynucleotide.

In some embodiments, the method further includes introducing the fifth polynucleotide into a fifth bacterium to obtain an expression vector component I bacterial library. In some embodiments, the method includes inserting the fifth polynucleotide into a component vector to form an expression vector fragment I storage ligation product, and introducing the storage ligation product into the fifth bacterium to obtain the expression vector component I bacterial library. In some embodiments, the method further includes acquiring a display fragment component plasmid I comprising the fifth polynucleotide from the expression vector component I bacterial library. In some embodiments, the method further includes acquiring the cleaved fifth polynucleotide from the display fragment component plasmid I. In some embodiments, the method includes digesting the display fragment component plasmid I with a restriction endonuclease that specifically recognizes the S6 and B2, thus obtaining the cleaved fifth polynucleotide.

In some embodiments, the method further includes introducing the sixth polynucleotide into a sixth bacterium to obtain an expression vector component II bacterial library. In some embodiments, the method includes inserting the sixth polynucleotide into a component vector to form an expression vector fragment II storage ligation product, and introducing the storage ligation product into the sixth bacterium to obtain the expression vector component II bacterial library. In some embodiments, the method further includes acquiring a display fragment component plasmid II comprising the sixth polynucleotide from the expression vector component II bacterial library. In some embodiments, the method further includes acquiring the cleaved sixth polynucleotide from the display fragment component plasmid II. In some embodiments, the method includes digesting the display fragment component plasmid II with a restriction endonuclease that specifically recognizes the B3 and B5, thus obtaining the cleaved sixth polynucleotide.

In some embodiments, the method further includes introducing the seventh polynucleotide into a seventh bacterium to obtain an expression vector component III bacterial library. In some embodiments, the method includes inserting the seventh polynucleotide into a component vector to form an expression vector fragment III storage ligation product, and introducing the storage ligation product into the seventh bacterium to obtain the expression vector component III bacterial library. In some embodiments, the method further includes acquiring a display fragment component plasmid III comprising the seventh polynucleotide from the expression vector component III bacterial library. In some embodiments, the method further includes acquiring the cleaved seventh polynucleotide from the display fragment component plasmid III. In some embodiments, the method includes digesting the display fragment component plasmid III with a restriction endonuclease that specifically recognizes the B6 and S5, thus obtaining the cleaved seventh polynucleotide.

In some embodiments, the method includes cryopreserving the LC1 light chain component bacterial library, the LC2 light chain component bacterial library, the VH1 heavy chain component bacterial library, the VH2 heavy chain component bacterial library, the expression vector component I bacterial library, the expression vector component II bacterial library and the expression vector component III bacterial library.

In some embodiments, the component vector is derived from a pUC vector.

In some embodiments, the pUC vector is a pUC19 vector or derived from a pUC19 vector.

In some embodiments, the LC1 light chain component bacterial library includes at least 10 different clones.

In some embodiments, the LC2 light chain component bacterial library includes at least 10 different clones.

In some embodiments, the VH1 heavy chain component bacterial library includes at least 10 different clones.

In some embodiments, the VH2 heavy chain component bacterial library includes at least 10 different clones.

In some embodiments, the bispecific antigen-binding polypeptide expression vector includes at least 10 different clones.

In some embodiments, the first polynucleotide, the second polynucleotide, the third polynucleotide and/or the fourth polynucleotide are obtained from sample materials.

In some embodiments, the sample materials include antibodies targeting specific antigens or antigen-binding fragments thereof.

In some embodiments, the antibodies or antigen-binding fragments thereof target PD-1 and/or PD-L1.

In some embodiments, the directional ligation involves using a ligase.

In some embodiments, the ligase includes T4 DNA ligase.

In another aspect, the present application provides a bispecific antigen-binding polypeptide expression vector produced by the method.

In another aspect, the present application provides a bispecific antigen-binding polypeptide display library established by utilizing the bispecific antigen-binding polypeptide expression vector.

In a certain embodiment, the library is a mammalian cell display library.

In a certain embodiment, the library is capable of displaying at least 10 different bispecific antibodies or antibody fragments thereof.

In another aspect, the present application provides a method for screening antibodies or antibody fragments, which involves utilizing the library of the present application.

Other aspects and advantages of the present application can be readily perceived by those skilled in the art from the following detailed description. In the following detailed description, only exemplary embodiments of the present application are shown and described. As will be recognized by those skilled in the art, the content of the present application enables those skilled in the art to make changes to the disclosed specific embodiments without departing from the spirit and scope of the invention involved in the present application. Correspondingly, the drawings and description in the specification of the present application are merely exemplary, rather than restrictive.

### Brief Description of the Drawings

The specific features of the invention involved in the present application are as shown in the appended claims. The characteristics and advantages of the invention involved in the present application can be better understood by referring to the exemplary embodiments described in detail below and the accompanying drawings. A brief description of the drawings is as below:
FIG. 1 shows the structure of the bispecific antigen-binding polypeptide expression vector of the present application;
FIG. 2 shows the structure of the bispecific antigen-binding polypeptide expression vector as a specific example in the present application;
FIG. 3 shows the expression of the bispecific antigen-binding polypeptide of the present application on the cell surface;
FIG. 4A-4J show that the bispecific antigen-binding polypeptide of the present application bind to the antigen in a dose-dependent manner.

### Detailed Description of the Embodiments

The implementation of the present application will be illustrated below by specific examples, and other advantages and effects of the present application will be easily known by those familiar with the art from the contents disclosed in the specification.

### Definition of Terms

In the present application, the term "storage ligation product" generally refers to a product form by ligating the digested polynucleotide with the nucleotide of the component vector. In the present application, the polynucleotide can be ligated with the component vector containing the recognition site for the same restriction endonuclease by ligase (e.g., DNA ligase) to obtain the storage ligation product.

In the present application, the term "component vector" generally refers to a nucleic acid molecule that may contain a target nucleic acid (such as, the first polynucleotide, the second polynucleotide, the third polynucleotide, the fourth polynucleotide, the fifth polynucleotide, the sixth polynucleotide and the seventh polynucleotide of the present application) and can be used for introducing the target nucleic acid into a cell. The term "vector" can include (but not limited to) plasmid, virus, cosmid and artificial chromosome. In general, an engineered vector can include an origin of replication, a restriction site and a selectable marker. The vector is generally a nucleotide sequence, usually a DNA sequence. In some cases, the component vector of the present application may be derived from a plasmid vector, for example, a pUC plasmid vector. For another example, the pUC vector may be a pUC19 vector or derived from a pUC19 vector.

In the present application, the term "introduction" generally refers to a process of inserting an exogenous polynucleotide into a cell, which may include "transfection", "transformation" or "transduction". "Introduction" can include introducing into a eukaryotic cell or a prokaryotic cell, that is, the nucleotide can enter the cell and be converted into an autonomous replicon. The cell may be a host cell. The introduced cell includes the primary cells of the subject and their progeny. The cell may be a prokaryotic cell, for example, it may be a bacterial cell.

In the present application, the term "antibody" generally refers to a polypeptide molecule capable of specifically recognizing and/or neutralizing a specific antigen. A basic 4-chain antibody unit is a heterotetrameric glycoprotein composed of two identical light chains and two identical heavy chains. Each heavy chain includes a heavy chain variable region (VH) and a heavy chain constant region. The heavy chain constant region is generally composed of three domains CH1, CH2 and CH3. Each light chain includes a light chain variable region (VL) and a light chain constant region. The light chain constant region includes one domain CL. The VH and VL regions can be further subdivided into multiple hypervariable regions, called complementary determining regions (CDRs), which are interspersed with more conserved regions called framework regions (FRs). Each of VH and VL is composed of three CDRs and four FRs, which are arranged from the amino terminus to the carboxy terminus in the following order: FR1, CDR1, FR2, CDR2, FR3, CDR3, FR4. The variable regions of heavy chain and light chain include binding domains interacting with the antigen.

In the present application, the term "bispecific binding polypeptide" generally refers to a polypeptide capable of specifically binding to at least two different antigens (e.g., a first target and a second target), which at least includes a first Fab for recognizing the first target and a second Fab for recognizing the second target.

In the present application, the term "Fab" generally refers to an antigen-binding fragment consisting of an intact L chain (which may include VL and CL) together with the variable region domain (VH) of one heavy chain and the first constant domain (CH1) of one heavy chain. Each Fab can have a single antigen binding site. The bispecific antigen-binding polypeptide can include the first Fab and the second Fab. The "first Fab" generally refers to the Fab that can recognize the first target of the bispecific antigen-binding polypeptide, which is generally formed by binding the first light chain to the first heavy chain variable region. The "second Fab" generally refers to the Fab that can recognize the second target of the bispecific antigen-binding polypeptide, which is generally formed by binding the second light chain to the second heavy chain variable region.

In the present application, the term "LC" generally refers to a polynucleotide comprising a nucleic acid sequence encoding the light chain or light chain fragment of the bispecific antigen-binding polypeptide. In the present application, the light chain or light chain fragment can have the ability of binding to the heavy chain of a same or similar antibody. In the present application, the light chain or light chain fragment can include a light chain variable region (VL) and a light chain constant region (CL). The light chain constant region can be divided into kappa-type and lambda-type. The light chain also includes a light chain having a lambda variable region (V-λ) linked to a kappa constant region (C-κ) or a kappa variable region (V-κ) linked to a lambda constant region (C-λ). The light chain of the present application can include an intact light chain and antigen-binding fragments thereof. Wherein, the polynucleotide encoding the light chain recognizing the first Fab of the first target in the bispecific antigen-binding polypeptide (that is, the first light chain) can be referred to as LC1, and the polynucleotide encoding the light chain recognizing the second Fab of the second target in the bispecific antigen-binding polypeptide (that is, the second light chain) can be referred to as LC2.

In the present application, the term "VH" generally refers to a polynucleotide comprising a nucleic acid sequence encoding the heavy chain variable region of the bispecific antigen-binding polypeptide. In the present application, the heavy chain variable region can have the ability of binding to the light chain or antigen-binding fragment thereof of a same or similar antibody. The heavy chain variable region can be a region including heavy chain (H) CDR1, frame (FR) 2, CDR2, FR3, CDR3 and FR4. The heavy chain variable region of the present application can include an intact heavy chain variable region and antigen-binding fragments thereof. Wherein, the polynucleotide encoding the heavy chain variable region recognizing the first Fab of the first target in the bispecific antigen-binding polypeptide (that is, the first heavy chain variable region) can be referred to as VH1, and the polynucleotide encoding the heavy chain variable region recognizing the second Fab of the second target in the bispecific antigen-binding polypeptide (that is, the second heavy chain variable region) can be referred to as VH2.

In the present application, the term "directional ligation" generally refers to the sequential ligation of different polynucleotides in one direction or one sequence. In the present application, the directional ligation of polynucleotides can be ensured by cleavage using a restriction endonuclease that specifically recognizes one cleavage site to form sticky ends that do not recognize or ligate to each other with restriction endonucleases that specifically recognize other cleavage sites. The directional ligation can involve using a ligase, e.g., DNA ligase.

In the present application, the term "polynucleotide" generally refers to at least two nucleotides linked together. The polynucleotides may be polymers of any length, including, for example, 10, 100, 200, 300, 500, 1000, 2000, 3000, 5000, 7000, 10,000, 100,000, etc. The polynucleotides can contain phosphodiester bonds. The "polynucleotide" may be any one of ribonucleotide or deoxyribonucleotide or modified forms of the two nucleotides. The polynucleotides of the present application may be linear.

In the present application, the term "cyclization" generally refers to a process of linking multiple polynucleotides end to end to form a circle. For example, the cleaved first polynucleotide, the cleaved second polynucleotide, the cleaved third polynucleotide, the cleaved fourth polynucleotide, the cleaved fifth polynucleotide, the cleaved sixth polynucleotide and the cleaved seventh polynucleotide in the present application can be cyclized to form the bispecific antigen-binding polypeptide expression vector.

In the present application, the term "clone" generally refers to the number of colonies. For example, the clone may be the number of colonies in the bacterial library (e.g., the LC1 light chain component bacterial library, the LC2 light chain component bacterial library, the VH1 heavy chain component bacterial library and/or the VH2 heavy chain component bacterial library) or in the expression vector. In some cases, the clone may be the number of different colonies in the bacterial library. In some cases, the clone may be the number of progeny populations produced by a single clone.

In the present application, the term "cleaved" polynucleotide generally refers to a polynucleotide with sticky ends produced after being treated with a restriction endonuclease.

In the present application, the term "restriction endonuclease" generally refers to an enzyme the cleaves double-stranded DNA. The restriction endonuclease can produce sticky ends with protruding single-stranded DNA that can bind to DNA ligase. In the present application, the restriction endonuclease can have the effects of recognition and restriction cleavage. For example, the cleavage site for the restriction endonuclease is at a certain distance from its recognition site. For example, the restriction endonuclease may be selected from SfiI and BsmBI.

In the present application, the term "restriction endonuclease specifically recognizing..." generally refers to a restriction endonuclease that can only recognize a polynucleotide containing a base sequence of a certain recognition site, and cannot recognize a polynucleotide containing a base sequence different from that of the recognition site.

In the present application, the term "specifically cleaving" generally means that only a polynucleotide containing a base sequence of a certain recognition site can be cleaved, and a polynucleotide containing a base sequence different from that of the recognition site cannot be cleaved.

In the present application, the term "corresponding restriction endonuclease" generally refers to restriction endonucleases capable of recognizing and cleaving the same nucleic acid sequence. The ends produced after recognition and cleavage by the corresponding restriction endonucleases are generally capable of recognizing or ligating each other.

In the present application, the term "display" generally refers to enabling the expression of the bispecific antigen-binding polypeptide in the cell comprising the bispecific antigen-binding polypeptide expression vector.

In the present application, the term "component plasmid" generally refers to a plasmid obtained from the bacteria in the bacterial library and comprising the polynucleotides (such as, the first polynucleotide, the second polynucleotide, the third polynucleotide, the fourth polynucleotide, the fifth polynucleotide, the sixth polynucleotide, and/or, the seventh polynucleotide). The component plasmid can also include a recognition site for the restriction endonuclease.

In the present application, the term "comprise" generally refers to the inclusion of explicitly specified features, but not excluding other elements.

In the present application, the term "about" generally refers to varying within a range of 0.5%-10% above or below the specified value, for example, varying within a range of 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, or 10% above or below the specified value.

### Detailed description

In one aspect, the present application provides a method for constructing a bispecific antigen-binding polypeptide expression vector.

### Acquisition of polynucleotides

The method can include providing polynucleotides, for example, the first polynucleotide, the second polynucleotide, the third polynucleotide, the fourth polynucleotide, the fifth polynucleotide, the sixth polynucleotide, and/or, the seventh polynucleotide.

### 1) Restriction site

The polynucleotide can include a recognition site for the restriction endonuclease. In the present application, the sequence of the recognition site for the restriction endonuclease is designed not to be included in the polynucleotide encoding the antigen-binding polypeptide or fragment thereof. The restriction endonucleases of the present application can specifically recognize S5, S6, B4, B3, B2, B5 and B6, respectively. Wherein, the B2, B3, B4, B5, B6, S5 and S6 can be each independently recognition sites for the restriction endonucleases.

The recognition sites for the restriction endonucleases in the present application can be specifically recognized by 1, 2, 3, 4, 5, 6, 7 or more restriction endonucleases, respectively. In some cases, the restriction endonucleases may be selected from SfiI and BsmBI. In other cases, other feasible restriction endonucleases can also be selected.

In some cases, the recognition sites for the restriction endonucleases may be the sites that are specifically recognized and cleaved by SfiI. For example, they can be referred to asS5 and S6, respectively. For example, the S5 can include a nucleic acid sequence as set forth in SEQ ID NO: 6. For another example, the S6 can include a nucleic acid sequence as set forth in SEQ ID NO: 7.

The recognition sites for the restriction endonucleases may be the sites that are specifically recognized and cleaved by BsmBI. For example, they can be referred to as B2, B3, B4, B5 and B6, respectively. For example, the B2 can include a nucleic acid sequence as set forth in SEQ ID NO: 1. Further for example, the B3 can include a nucleic acid sequence as set forth in SEQ ID NO: 2. For another example, the B4 can include a nucleic acid sequence as set forth in SEQ ID NO: 3. For another example, the B5 can include a nucleic acid sequence as set forth in SEQ ID NO: 4. For another example, the B6 can include a nucleic acid sequence as set forth in SEQ ID NO: 5.

It should be noted that, the recognition sites for the restriction endonucleases in the present application include, but are not limited to, the recognition sites listed herein, and may also include the recognition sites for other restriction endonucleases not listed, as well as other recognition sites for the restriction endonucleases, provided that they do not cause undesired recognition or cleavage of the target sequence (e.g., a polynucleotide encoding the antigen-binding polypeptide or fragments thereof).

### 2) Bispecific antigen-binding polypeptide

The polynucleotides of the present application (e.g., the first polynucleotide, the second polynucleotide, the third polynucleotide, the fourth polynucleotide, the fifth polynucleotide, the sixth polynucleotide and the seventh polynucleotide) may also include polynucleotide LC1, VH1, LC2, VH2 encoding the bispecific antigen-binding polypeptide or fragments thereof.

In the present application, the LC1 can encode a first light chain of the bispecific antigen-binding polypeptide, the VH1 can encode a first heavy chain variable region of the bispecific antigen-binding polypeptide, and the first light chain can bind to the first heavy chain variable region to form a first Fab for recognizing a first target. In the present application, the LC2 can encode a second light chain of the bispecific antigen-binding polypeptide, the VH2 can encode a second heavy chain variable region of the bispecific antigen-binding polypeptide, and the second light chain can bind to the second the heavy chain variable region to form a second Fab for recognizing a second target. In some cases, the first target and second target may be antigens.

In the present application, the first Fab can bind to the second Fab to form the bispecific antigen-binding polypeptide.

The polynucleotides of the present application (e.g., the first polynucleotide, the second polynucleotide, the third polynucleotide, the fourth polynucleotide, the fifth polynucleotide, the sixth polynucleotide and the seventh polynucleotide) can include expression vector fragments, such as, the expression vector fragment I, the expression vector fragment II and the expression vector fragment III. The desired length or type of the expression vector fragment I, the expression vector fragment II, and the expression vector fragment III can be selected respectively according to the length or nature of the bispecific antigen-binding polypeptide or fragments thereof to be expressed, and the length or nature of the restriction sites.

In some cases, the expression vector fragment I, the expression vector fragment II and the expression vector fragment III may be derived from the vector fragment of any one vector capable of expressing the target gene. For example, the expression vector fragment I, the expression vector fragment II and the expression vector fragment III may be derived from the fragments of the display vector pDGB4 (with regard to pDGB4, see Ivan Zhou, et al., "Four-way ligation for construction of a mammalian cell-based full-length antibody display library", Acta Biochim Biophys Sin 2011, 43: 232-238).

The expression vector fragments of the present application (e.g., the expression vector fragment I, the expression vector fragment II and the expression vector fragment III) can include nucleotide sequences with specific functions, including, but not limited to, promoters, enhancers, signal peptides, screening markers (for example, they may include enzyme recognition sites, resistance genes, reporter genes, and screening genes), which can be adjusted in the expression vector fragments by those skilled in the art according to the desired function ( inserting/substituting and/or deleting the above nucleotide sequences with specific functions). In some cases, the expression vector fragments can be adjusted in different cases to get different nucleotide sequences.

In the present application, the first polynucleotide can include S5-LC1-S6 in the direction from 5' to 3', wherein, S5 and S6 can be each independently recognition sites for the restriction endonuclease, the LC1 can encode a first light chain of the bispecific antigen-binding polypeptide. In some cases, the S5 and S6 can be specifically recognized and cleaved by Sfil, respectively. For example, the S5 can include a nucleic acid sequence as set forth in SEQ ID NO: 6, and the S5 can include a nucleic acid sequence as set forth in SEQ ID NO: 7.

The second polynucleotide can include B4-VH1-B3 in the direction from 5' to 3', wherein, B4 and B3 can be each independently recognition sites for the restriction endonuclease, the VH1 can encode a first heavy chain variable region of the bispecific antigen-binding polypeptide. In some cases, the B4 and B3 can be specifically recognized and cleaved by BsmBI, respectively. For example, the B4 can include a nucleic acid sequence as set forth in SEQ ID NO: 3, and the B3 can include a nucleic acid sequence as set forth in SEQ ID NO: 2.

The third polynucleotide can include B2-LC2-B4 in the direction from 5' to 3', wherein, B2 and B4 can be each independently recognition sites for the restriction endonuclease, the LC2 can encode a second light chain of the bispecific antigen-binding polypeptide. In some cases, the B2 and B4 can be specifically recognized and cleaved by BsmBI, respectively. For example, the B2 can include a nucleic acid sequence as set forth in SEQ ID NO: 1, and the B4 can include a nucleic acid sequence as set forth in SEQ ID NO: 3.

The fourth polynucleotide can include B5-VH2-B6 in the direction from 5' to 3', wherein, B5 and B6 can be each independently recognition sites for the restriction endonuclease, the VH2 can encode a second heavy chain variable region of the bispecific antigen-binding polypeptide. In some cases, the B5 and B6 can be specifically recognized and cleaved by BsmBI, respectively. For example, the B5 can include a nucleic acid sequence as set forth in SEQ ID NO: 4, and the B6 can include a nucleic acid sequence as set forth in SEQ ID NO: 7.

The fifth polynucleotide can include S6-expression vector fragment I-B2 in the direction from 5' to 3', wherein, S6 and B2 can be each independently recognition sites for the restriction endonuclease. In some cases, the S6 can be specifically recognized and cleaved by Sfil, the B3 can be specifically recognized and cleaved by BsmBI. For example, the S6 can include a nucleic acid sequence as set forth in SEQ ID NO: 7, and the B2 can include a nucleic acid sequence as set forth in SEQ ID NO: 1.

The sixth polynucleotide can include B3-expression vector fragment II-B5 in the direction from 5' to 3', wherein, B3 and B5 can be each independently recognition sites for the restriction endonuclease. In some cases, the B3 and B5 can be specifically recognized and cleaved by BsmBI, respectively. For example, the B3 can include a nucleic acid sequence as set forth in SEQ ID NO: 2, and the B5 can include a nucleic acid sequence as set forth in SEQ ID NO: 4.

The seventh polynucleotide can include B6-expression vector fragment III-S5 in the direction from 5' to 3', wherein, B6 and S5 can be each independently recognition sites for the restriction endonuclease. In some cases, the B6 can be specifically recognized and cleaved by BsmBI, and the S5 can be specifically recognized and cleaved by Sfil. For example, the B6 can include a nucleic acid sequence as set forth in SEQ ID NO: 5, and the S5 can include a nucleic acid sequence as set forth in SEQ ID NO: 6.

The first polynucleotide, the second polynucleotide, the third polynucleotide and/or the fourth polynucleotide of the present application can be obtained from sample materials. In some cases, the sample materials can include antibodies targeting specific antigens or antigen-binding fragments thereof. The antigens may be any immunogenic fragments or determinants, including, but not limited to, PD-1, PD-L1, LAG-3, CD47, CD3. For example, the antibodies or antigen-binding fragments thereof target PD-1 and/or PD-L1.

The method of the present application can include introducing the polynucleotides (e.g., the first polynucleotide, the second polynucleotide, the third polynucleotide, the fourth polynucleotide, the fifth polynucleotide, the sixth polynucleotide and the seventh polynucleotide) into bacteria.

In order to screen positive bacteria into which the polynucleotides have been introduced, the polynucleotides (e.g., ) can also include nucleic acid sequences encoding signal peptides, for example, signal peptides expressing natural resistance genes. In one example, the 3'-end of the nucleic acid sequence encoding a signal peptide can bind to the restriction site at the 5'-end of the polynucleotide. In some cases, in order to introduce a suitable restriction site to the 3'-end portion of the nucleic acid sequence encoding a signal peptide, its base sequence can be changed by unintentional mutation, but the amino acid sequence of the signal peptide remains unchanged. For example, the nucleic acid sequence encoding the signal peptide can include a nucleic acid sequence as set forth in any one selected from SEQ ID NO: 8, SEQ ID NO: 10, and SEQ ID NO: 12; alternatively, the signal peptide can include an amino acid sequence as set forth in any one selected from SEQ ID NO: 9, SEQ ID NO: 11, and SEQ ID NO: 13.

The polynucleotides can be obtained by conventional methods in the art, which can include, but not limited to: standard PCR, long PCR, hot start PCR, qPCR, RT-PCR and isothermal amplification. In some cases, primers can be designed according to the sequences of the target fragments (e.g., LC1, VH1, LC2, VH2, the expression vector fragment I, the expression vector fragment II and the expression vector fragment III), respectively, which were then used as templates for amplification to obtain the polynucleotides. For example, the primer for amplifying the LC1 can include a nucleotide sequence as set forth in any one selected from SEQ ID NO: 20 and SEQ ID NO: 21. For example, the primer for amplifying the LC2 can include a nucleotide sequence as set forth in any one selected from SEQ ID NO: 22 and SEQ ID NO: 23. For example, the primer for amplifying the VH1 can include a nucleotide sequence as set forth in any one selected from SEQ ID NO: 24 and SEQ ID NO: 25. For example, the primer for amplifying the VH2 can include a nucleotide sequence as set forth in any one selected from SEQ ID NO: 26 and SEQ ID NO: 27. For example, the primer for amplifying the expression vector fragment I can include a nucleotide sequence as set forth in any one selected from SEQ ID NO: 14 and SEQ ID NO: 15. For example, the primer for amplifying the expression vector fragment II can include a nucleotide sequence as set forth in any one selected from SEQ ID NO: 16 and SEQ ID NO: 17. For example, the primer for amplifying the expression vector fragment III can include a nucleotide sequence as set forth in any one selected from SEQ ID NO: 18 and SEQ ID NO: 19.

### Establishment of bacterial library

After the polynucleotides were obtained, they can be separately introduced into a bacterium to obtain a bacterial library. Therefore, the method of the present application can further include the following steps: introducing the first polynucleotide into a first bacterium to obtain an LC1 light chain component bacterial library; introducing the second polynucleotide into a second bacterium to obtain a VH1 heavy chain component bacterial library; introducing the third polynucleotide into a third bacterium to obtain an LC2 light chain component bacterial library; introducing the fourth polynucleotide into a fourth bacterium to obtain a VH2 heavy chain component bacterial library; introducing the fifth polynucleotide into a fifth bacterium to obtain an expression vector component I bacterial library; introducing the sixth polynucleotide into a sixth bacterium to obtain an expression vector component II bacterial library; and introducing the seventh polynucleotide into a seventh bacterium to obtain an expression vector component III bacterial library.

In some cases, the polynucleotides can be inserted into the component vectors to form storage ligation products. In some cases, the polynucleotides can be inserted into the component vectors by using PCR cloning. The component vectors can include plasmid vectors (e.g., pBR322, pUC vectors), phage vectors (e.g., M13 vector, λ vector), phage-derived plasmids (e.g., phagemid, cosmid), and bacterial artificial chromosome (BAC). In some embodiments, the component vector may be derived from a pUC vector. For example, the component vector may be a pUC19 vector or derived from a pUC19 vector.

Then, the storage ligation products can be introduced into the bacterium to obtain the bacterial library.

In the present application, the method can include inserting the first polynucleotide into a component vector to form an LC1 storage ligation product, and introducing the LC1 storage ligation product into the first bacterium to obtain an LC1 light chain component bacterial library. In some cases, the LC1 light chain component bacterial library can include at least 10 (e.g., at least 10, at least 11, at least 12, at least 13, at least 14, at least 15, at least 16, at least 17, at least 18, at least 19, at least 20, at least 25, at least 30, at least 35, at least 40, at least 45, at least 50, or more) different clones.

The method can include inserting the third polynucleotide into a component vector to form an LC2 storage ligation product, and introducing the LC2 storage ligation product into the third bacterium to obtain the LC2 light chain component bacterial library. In some cases, the LC2 light chain component bacterial library can include at least 10 (e.g., at least 10, at least 11, at least 12, at least 13, at least 14, at least 15, at least 16, at least 17, at least 18, at least 19, at least 20, at least 25, at least 30, at least 35, at least 40, at least 45, at least 50, or more) different clones.

The method can include inserting the second polynucleotide into a component vector to form a VH1 storage ligation product, and introducing the VH1 storage ligation product into the second bacterium to obtain the VH1 heavy chain component bacterial library. In some cases, the VH1 heavy chain component bacterial library can include at least 10 (e.g., at least 10, at least 11, at least 12, at least 13, at least 14, at least 15, at least 16, at least 17, at least 18, at least 19, at least 20, at least 25, at least 30, at least 35, at least 40, at least 45, at least 50, or more) different clones.

The method can include inserting the fourth polynucleotide into a component vector to form a VH2 storage ligation product, and introducing the VH2 storage ligation product into the fourth bacterium to obtain the VH2 heavy chain component bacterial library. In some cases, the VH2 heavy chain component bacterial library can include at least 10 (e.g., at least 10, at least 11, at least 12, at least 13, at least 14, at least 15, at least 16, at least 17, at least 18, at least 19, at least 20, at least 25, at least 30, at least 35, at least 40, at least 45, at least 50, or more) different clones.

The method can include inserting the fifth polynucleotide into a component vector to form an expression vector fragment I storage ligation product, and introducing the storage ligation product into the fifth bacterium to obtain the expression vector component I bacterial library. In some cases, the expression vector component I bacterial library can include at least 10 (e.g., at least 10, at least 11, at least 12, at least 13, at least 14, at least 15, at least 16, at least 17, at least 18, at least 19, at least 20, at least 25, at least 30, at least 35, at least 40, at least 45, at least 50, or more) different clones.

The method can include inserting the sixth polynucleotide into a component vector to form an expression vector fragment II storage ligation product, and introducing the storage ligation product into the sixth bacterium to obtain the expression vector component II bacterial library. In some cases, the expression vector component II bacterial library can include at least 10 (e.g., at least 10, at least 11, at least 12, at least 13, at least 14, at least 15, at least 16, at least 17, at least 18, at least 19, at least 20, at least 25, at least 30, at least 35, at least 40, at least 45, at least 50, or more) different clones.

The method can include inserting the seventh polynucleotide into a component vector to form an expression vector fragment III storage ligation product, and introducing the storage ligation product into the seventh bacterium to obtain the expression vector component III bacterial library. In some cases, the expression vector component III bacterial library can include at least 10 (e.g., at least 10, at least 11, at least 12, at least 13, at least 14, at least 15, at least 16, at least 17, at least 18, at least 19, at least 20, at least 25, at least 30, at least 35, at least 40, at least 45, at least 50, or more) different clones.

The method of the present application may further include cryopreserving the LC1 light chain component bacterial library, the LC2 light chain component bacterial library, the VH1 heavy chain component bacterial library, the VH2 heavy chain component bacterial library, the expression vector component I bacterial library, the expression vector component II bacterial library, and the expression vector component III bacterial library.

In the present application, the method can include obtaining a component plasmid comprising the polynucleotide from the bacterial library after obtaining the bacterial library.

In some cases, the method can include acquiring a first light chain component plasmid comprising the first polynucleotide from the LC1 light chain component bacterial library, where the first light chain component plasmid may also include the S5 and the S6. In some cases, the method can include acquiring a second light chain component plasmid comprising the third polynucleotide from the LC2 light chain component bacterial library, where the second light chain component plasmid may also include the B2 and the B4. In some cases, the method can include acquiring a first light chain component plasmid comprising the second polynucleotide from the VH1 heavy chain component bacterial library, where the first heavy chain component plasmid may also include the B4 and the B3. In some cases, the method can include acquiring a second heavy chain component plasmid comprising the fourth polynucleotide from the VH2 heavy chain component bacterial library, where the second heavy chain component plasmid may also include the B5 and the B6. In some cases, the method can include acquiring a display fragment component plasmid I comprising the fifth polynucleotide from the expression vector component I bacterial library, where the display fragment component plasmid I may also include the S6 and the B2. In some cases, the method can include acquiring a display fragment component plasmid II comprising the sixth polynucleotide from the expression vector component II bacterial library, where the display fragment component plasmid II may also include the B3 and the B5. In some cases, the method can include acquiring a display fragment component plasmid III comprising the seventh polynucleotide from the expression vector component III bacterial library, where the display fragment component plasmid III may also include the B6 and the S5.

### Acquisition of cleaved polynucleotides

The method of the present application may further include h) specifically cleaving the first polynucleotide, the second polynucleotide, the third polynucleotide, the fourth polynucleotide, the fifth polynucleotide, the sixth polynucleotide and the seventh polynucleotide with a restriction endonuclease to obtain a cleaved first polynucleotide, a cleaved second polynucleotide, a cleaved third polynucleotide, a cleaved fourth polynucleotide, a cleaved fifth polynucleotide, a cleaved sixth polynucleotide, and a cleaved seventh polynucleotide.

In some cases, after obtaining the component plasmid comprising the polynucleotide, the method may further include acquiring the cleaved polynucleotide from the component plasmid. In some cases, the plasmid can be digested with the restriction endonuclease to obtain the cleaved polynucleotide. The method can include the steps of: acquiring the cleaved first polynucleotide from the first light chain component plasmid; acquiring the cleaved second polynucleotide from the second light chain component plasmid; acquiring the cleaved third polynucleotide from the first heavy chain component plasmid; acquiring the cleaved fourth polynucleotide from the second heavy chain component plasmid; acquiring the cleaved fifth polynucleotide from the display fragment component plasmid I; acquiring the cleaved sixth polynucleotide from the display fragment component plasmid II; and acquiring the cleaved seventh polynucleotide from the display fragment component plasmid III.

In some cases, the first light chain component plasmid can be digested with a restriction endonuclease that specifically recognizes the S5 and S6, thus obtaining the cleaved first polynucleotide.

In some cases, the first heavy chain component plasmid can be digested with a restriction endonuclease that specifically recognizes the B4 and B3, thus obtaining the cleaved second polynucleotide.

In some cases, the second light chain component plasmid can be digested with a restriction endonuclease that specifically recognizes the B2 and B4, thus obtaining the cleaved third polynucleotide.

In some cases, the display fragment component plasmid I can be digested with a restriction endonuclease that specifically recognizes the S6 and B2, thus obtaining the cleaved fifth polynucleotide.

In some cases, the display fragment component plasmid II can be digested with a restriction endonuclease that specifically recognizes the B3 and B5, thus obtaining the cleaved sixth polynucleotide.

In some cases, the display fragment component plasmid III can be digested with a restriction endonuclease that specifically recognizes the B6 and S5, thus obtaining the cleaved seventh polynucleotide.

After being digested with a restriction endonuclease, sticky ends can be produced at the 3'-end and/or 5'-end of the component plasmid.

In some cases, the end produced from the specific cleavage of B2 by the restriction endonuclease that specifically recognizes it does not recognize or link to each other with the end produced from the specific cleavage of any one of the B3, B4, B5, B6, S5 and S6 by the corresponding restriction endonuclease. Alternatively, the end produced after specific cleavage by a restriction endonuclease that can specifically recognize B2 can only recognize or link to each other with the end produced after specific cleavage by a restriction endonuclease that can specifically recognize B2.

In some cases, the end produced from the specific cleavage of B3 by the restriction endonuclease that specifically recognizes it does not recognize or link to each other with the end produced from the specific cleavage of any one of the B2, B4, B5, B6, S5 and S6 by the corresponding restriction endonuclease. Alternatively, the end produced after specific cleavage by a restriction endonuclease that can specifically recognize B3 can only recognize or link to each other with the end produced after specific cleavage by a restriction endonuclease that can specifically recognize B3.

In some cases, the end produced from the specific cleavage of B4 by the restriction endonuclease that specifically recognizes it does not recognize or link to each other with the end produced from the specific cleavage of any one of the B2, B3, B5, B6, S5 and S6 by the corresponding restriction endonuclease. Alternatively, the end produced after specific cleavage by a restriction endonuclease that can specifically recognize B4 can only recognize or link to each other with the end produced after specific cleavage by a restriction endonuclease that can specifically recognize B4.

In some cases, the end produced from the specific cleavage of B5 by the restriction endonuclease that specifically recognizes it does not recognize or link to each other with the end produced from the specific cleavage of any one of the B2, B4, B3, B6, S5 and S6 by the corresponding restriction endonuclease. Alternatively, the end produced after specific cleavage by a restriction endonuclease that can specifically recognize B5 can only recognize or link to each other with the end produced after specific cleavage by a restriction endonuclease that can specifically recognize B5.

In some cases, the end produced from the specific cleavage of B6 by the restriction endonuclease that specifically recognizes it does not recognize or link to each other with the end produced from the specific cleavage of any one of the B2, B4, B5, B3, S5 and S6 by the corresponding restriction endonuclease. Alternatively, the end produced after specific cleavage by a restriction endonuclease that can specifically recognize B6 can only recognize or link to each other with the end produced after specific cleavage by a restriction endonuclease that can specifically recognize B6.

In some cases, the end produced from the specific cleavage of S5 by the restriction endonuclease that specifically recognizes it does not recognize or link to each other with the end produced from the specific cleavage of any one of the B2, B4, B5, B6, B3 and S6 by the corresponding restriction endonuclease. Alternatively, the end produced after specific cleavage by a restriction endonuclease that can specifically recognize S5 can only recognize or link to each other with the end produced after specific cleavage by a restriction endonuclease that can specifically recognize S5.

In some cases, the end produced from the specific cleavage of S6 by the restriction endonuclease that specifically recognizes it does not recognize or link to each other with the end produced from the specific cleavage of any one of the B2, B4, B5, B6, B3 and S5 by the corresponding restriction endonuclease. Alternatively, the end produced after specific cleavage by a restriction endonuclease that can specifically recognize S6 can only recognize or link to each other with the end produced after specific cleavage by a restriction endonuclease that can specifically recognize S6.

### Acquisition of the bispecific antigen-binding polypeptide expression vector by ligation

In the present application, the method may further include: i) mixing the cleaved first polynucleotide, the cleaved second polynucleotide, the cleaved third polynucleotide, the cleaved fourth polynucleotide, the cleaved fifth polynucleotide, the cleaved sixth polynucleotide, and the cleaved seventh polynucleotide, so that they can be ligated directionally and cyclized to form the bispecific antigen-binding polypeptide expression vector. For example, the cleaved polynucleotides can be mixed in equal proportions, and introduced into a cell (e.g., a mammalian cell). The colonies are then sorted for sequencing to determine the expression vector containing the desired bispecific antigen-binding polypeptide sequence.

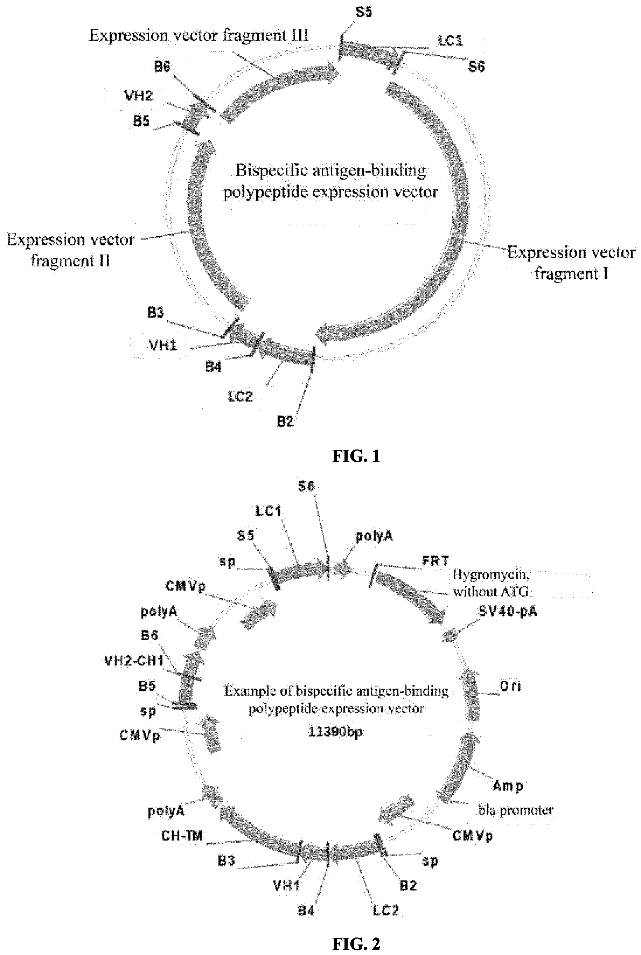

For example, the structure of the expression vector can be as shown in FIG. 1, which is formed by directional ligation and cyclization of the seven cleaved polynucleotides. The end at the 3'-end of the first polynucleotide after specific cleavage by a restriction endonuclease that specifically recognizes S6 can recognize or link to each other with the end at the 5'-end of the fifth polynucleotide after specific cleavage by a restriction endonuclease that specifically recognizes S6. The end at the 3'-end of the fifth polynucleotide after specific cleavage by a restriction endonuclease that specifically recognizes B2 can recognize or link to each other with the end of the third polynucleotide after specific cleavage by a restriction endonuclease that specifically recognizes B2. The end at the 3'-end of the third polynucleotide after specific cleavage by a restriction endonuclease that specifically recognizes B4 can recognize or link to each other with the end at the 5'-end of the second polynucleotide after specific cleavage by a restriction endonuclease that specifically recognizes B4. The end at the 3'-end of the second polynucleotide after specific cleavage by a restriction endonuclease that specifically recognizes B3 can recognize or link to each other with the end at the 5'-end of the sixth polynucleotide after specific cleavage by a restriction endonuclease that specifically recognizes B3. The end at the 3'-end of the sixth polynucleotide after specific cleavage by a restriction endonuclease that specifically recognizes B5 can recognize or link to each other with the end at the 5'-end of the fourth polynucleotide after specific cleavage by a restriction endonuclease that specifically recognizes B5. The end at the 3'-end of the fourth polynucleotide after specific cleavage by a restriction endonuclease that specifically recognizes B6 can recognize or link to each other with the end at the 5'-end of the seventh polynucleotide after specific cleavage by a restriction endonuclease that specifically recognizes B6. The end at the 3'-end of the seventh polynucleotide after specific cleavage by a restriction endonuclease that specifically recognizes S5 can recognize or link to each other with the end at the 5'-end of the first polynucleotide after specific cleavage by a restriction endonuclease that specifically recognizes S5.

In some cases, the directional ligation can involve using a ligase. For example, the ligase can include T4 DNA ligase.

The bispecific antigen-binding polypeptide expression vector can include at least 10 (e.g., at least 10, at least 11, at least 12, at least 13, at least 14, at least 15, at least 16, at least 17, at least 18, at least 19, at least 20, at least 25, at least 30, at least 35, at least 40, at least 45, at least 50, or more) different clones.

In another aspect, the present application provides a bispecific antigen-binding polypeptide expression vector produced according to the described method. The bispecific antigen-binding polypeptide expression vector can include at least 10 (e.g., at least 10, at least 11, at least 12, at least 13, at least 14, at least 15, at least 16, at least 17, at least 18, at least 19, at least 20, at least 25, at least 30, at least 35, at least 40, at least 45, at least 50, or more) different clones.

In another aspect, the present application provides a bispecific antigen-binding polypeptide display library established by utilizing the bispecific antigen-binding polypeptide expression vector. In some embodiments, the display library may be a mammalian cell display library. In some embodiments, the library can display at least 10 (e.g., at least 10, at least 11, at least 12, at least 13, at least 14, at least 15, at least 16, at least 17, at least 18, at least 19, at least 20, at least 25, at least 30, at least 35, at least 40, at least 45, at least 50, or more) different bispecific antibodies or antibody fragments thereof.

In another aspect, the present application provides a method for screening antibodies or antibody fragments, which can involve using the library of the present application. In some embodiments, the display library may be a mammalian cell display library. In some embodiments, the library can display at least 10 (e.g., at least 10, at least 11, at least 12, at least 13, at least 14, at least 15, at least 16, at least 17, at least 18, at least 19, at least 20, at least 25, at least 30, at least 35, at least 40, at least 45, at least 50, or more) different bispecific antibodies or antibody fragments thereof. In some cases, the antibodies or antibody fragments may be bispecific antigen-binding polypeptides. For example, the method can include: selecting mammalian cells from the library, establishing a cell line stably expressing the bispecific antigen-binding polypeptide, and then screening. For example, the expression of the bispecific antigen-binding polypeptide on the cell surface and its specific affinity for at least two antigens can be analyzed using FACS.

Without intending to be limited by any theory, the following examples are only to illustrate the fusion protein, its preparation method and use of the present application, and are not used to limit the inventive scope of the present application.

### Example

### Example 1 Construction of bispecific antigen-binding polypeptide expression vector

### 1.1 Acquisition of sample materials

In order to construct the bispecific antigen-binding polypeptide expression vector as shown in FIG. 2, a PD-1-targeting antibody Pembrolizumab and a PD-L1-targeting antibody Atezolizumab, as well as a pDGB4 vector were chosen as examples. The nucleotide sequence of the light chain of Pembrolizumab was: SEQ ID NO: 28, the nucleotide sequence of the heavy chain variable region of Pembrolizumab was: SEQ ID NO: 29, the nucleotide sequence of the light chain of Atezolizumab was: SEQ ID NO: 30, the nucleotide sequence of the heavy chain variable region of Atezolizumab was: SEQ ID NO: 31, and the nucleotide sequence of the pDGB4 vector was: SEQ ID NO: 32.

### 1.2 Design of restriction site

According to the restriction endonucleases BsmBI and SfiI, the sequences of 5 BsmBI recognition sites (B2, B3, B4, B5 and B6) and the sequences of 2 SfiI recognition sites (S5 and S6) were designed, with the sequences shown in Table 1 below:

**Table 1**

| Restriction site | Nucleic acid sequence |
|---|---|
| B2 | SEQ ID NO: 1 |
| B3 | SEQ ID NO: 2 |
| B4 | SEQ ID NO: 3 |
| B5 | SEQ ID NO: 4 |
| B6 | SEQ ID NO: 5 |
| S5 | SEQ ID NO: 6 |
| S6 | SEQ ID NO: 7 |

### 1.3 Selection of signal peptides

Three signal peptides expressing native antibody genes were selected, wherein, sp1 was located at the 5'-end of LC2, sp2 was located at the 5'-end of VH2, and sp3 was located at the 5'-end of LC1. In order to introduce a suitable restriction site to the 3'-end part of the nucleic acid sequence encoding a signal peptide, the base sequences of the three encoded signal peptides have been changed by unintentional mutation, but the amino acid sequences of the signal peptides remained unchanged. The sequences were shown in Table 2 below:

**Table 2**

| Signal peptide | Sequence |
|---|---|
| sp1 nucleic acid sequence | SEQ ID NO: 8 |
| sp1 amino acid sequence | SEQ ID NO: 9 |
| sp2 nucleic acid sequence | SEQ ID NO: 10 |
| sp2 amino acid sequence | SEQ ID NO: 11 |
| sp3 nucleic acid sequence | SEQ ID NO: 12 |
| sp3 amino acid sequence | SEQ ID NO: 13 |

### 1.4 Acquisition of polynucleotides

Primers were designed for the light chain (LC1) and heavy chain variable region sequence (VH1) encoding PD-1, the light chain (LC2) and heavy chain variable region (VH2) of PD-L1, and the nucleic acid sequence of the three-segment vector pDGB4 (the three segments were respectively the expression vector fragment I, the expression vector fragment II and the expression vector fragment III), respectively, with the 5-ends of the primers containing the restriction sites. In addition, the expression frameworks for LC1, LC2-VH1, and LC2 were all driven by the CMV promoter for expression. The primers were synthesized, with the sequences shown in Table 3.

**Table 3**

| Polynucleotide | Template | Forward primer | Reverse primer |
|---|---|---|---|
| LC1 | PD1-LC (SEQ ID NO: 28) | P7(SEQ ID NO: 20) | P8(SEQ ID NO: 21) |
| VH1 | PD1-VH (SEQ ID NO:29) | P11(SEQ ID NO: 24) | P12(SEQ ID NO: 25) |
| LC2 | PDL1-LC (SEQ ID NO: 30) | P9(SEQ ID NO: 22) | P10(SEQ ID NO: 23) |
| VH2 | PDL1-VH (SEQ ID NO: 31) | P13(SEQ ID NO: 26) | P14(SEQ ID NO: 26) |
| Expression vector fragment I | pDGB4 (SEQ ID NO: 32) | P1(SEQ ID NO: 14) | P2(SEQ ID NO: 15) |
| Expression vector fragment II | pDGB4 (SEQ ID NO: 32) | P3(SEQ ID NO: 16) | P4(SEQ ID NO: 17) |
| Expression vector fragment III | pDGB4 (SEQ ID NO: 32) | P5(SEQ ID NO:18) | P6(SEQ ID NO:19) |

Seven polynucleotides were amplified by PCR (LA Taq, Takara Co., performed according to the product instruction of the company), and the template and primer sequences used were shown in Table 3. PCR products were obtained respectively after purification and recovery by gel electrophoresis (operated according to the instruction in "Molecular Cloning: A Laboratory Manual"). By means of the method of TA cloning (TA cloning kit, purchased from Takara Co.), the PCR products were inserted into a pUC19 plasmid vector to obtain the storage ligation product. The DH5a competent bacteria (Takara Co.) were transformed with the storage vector product, and cultured overnight at 37°C on a plate. The colonies were sent for sequencing, and then bacteria were obtained which are polynucleotides containing the desired sequences, that are the first polynucleotide containing LC1, the second polynucleotide containing VH1, the third polynucleotide containing LC2, the fourth polynucleotide containing VH2, the fifth polynucleotide containing the expression vector fragment I, the sixth polynucleotide containing the expression vector fragment II, and the seventh polynucleotide containing the expression vector fragment III. The bacteria can be cryopreserved as the bacterial library for later use.

### 1.5 Digestion

The plasmids of bacteria in the bacterial library of Example 1.4 were respectively extracted by using a plasmid extraction kit (purchased from Axygen). Then, the plasmid vectors were digested with restriction endonucleases BsmBI and SfiI, the first polynucleotide was cleaved with a restriction endonuclease that specifically recognizes S5 and S6, the second polynucleotide was cleaved with a restriction endonuclease that specifically recognizes B4 and B3, the third polynucleotide was cleaved with a restriction endonuclease that specifically recognizes B2 and B4, the fourth polynucleotide was cleaved with a restriction endonuclease that specifically recognizes B5 and B6, the fifth polynucleotide was cleaved with a restriction endonuclease that specifically recognizes S6 and B2, the sixth polynucleotide was cleaved with a restriction endonuclease that specifically recognizes B3 and B5, the seventh polynucleotide was cleaved with a restriction endonuclease that specifically recognizes B6 and S5. Isolation and purification were performed by electrophoresis to obtain the seven cleaved polynucleotides.

### 1.6 Acquisition of expression vectors

The seven cleaved polynucleotides obtained in Example 1.5 were mixed in equal molecular proportions, into which was added a ligase so that they were ligated directionally and cyclized to form an expression vector. The expression vector was transferred into DH5a competent bacteria (Takara, operated according to the instruction of the manufacturer), and cultured in 2YT medium without antibiotics at 37°C while shaking at 250 rpm for 60 minutes, then spread on ampicillin-resistant plates (Thermo, Cat#240845) and grown overnight at 37°C. The colonies were sorted for sequencing, and expression vectors containing correct sequences were obtained. The expression vectors were transferred into FCHO cells to establish cell lines stably expressing the bispecific antigen-binding polypeptides, thereby obtaining a cell display library.

### Example 2 Expression of the bispecific antigen-binding polypeptides on the cell surface

The cells from the cell library of Example 1.6 were adherently cultured. After reaching a certain concentration, the dispersed cells were digested with 0.5 mM EDTA-PBS, and collected by centrifugation. The collected cells were suspended with staining buffer (2% FBS-PBS), and distributed into a 96-well plate at 5e4 cells/50 ul per well. Into the wells were added 1-3 kinds of fluorescently labeled antibodies or antigens listed below according to the experimental requirements, mixed well, and incubated on ice for 30 minutes. After then, staining buffer was added at 200 ul/well, and flow cytometry was then performed. The wells without fluorescently labeled antigens/antibodies added were set as the negative control.

PEK: PE-labeled mouse anti-human Kappa light chain antibody, which was detected whether there was a Kappa light chain on the cell surface; FITC-G: FITC-labeled mouse anti-human IgG heavy chain antibody, which was detected whether there was an IgG heavy chain on the cell surface; FITC-Ag1: FITC-labeled PD1 antigen, which was detected whether the antibody displayed on the cell surface could bind to the PD1 antigen; FITC-Ag2: FITC-labeled PDL1 antigen, which was detected whether the antibody displayed on the cell surface could bind to the PDL1 antigen.

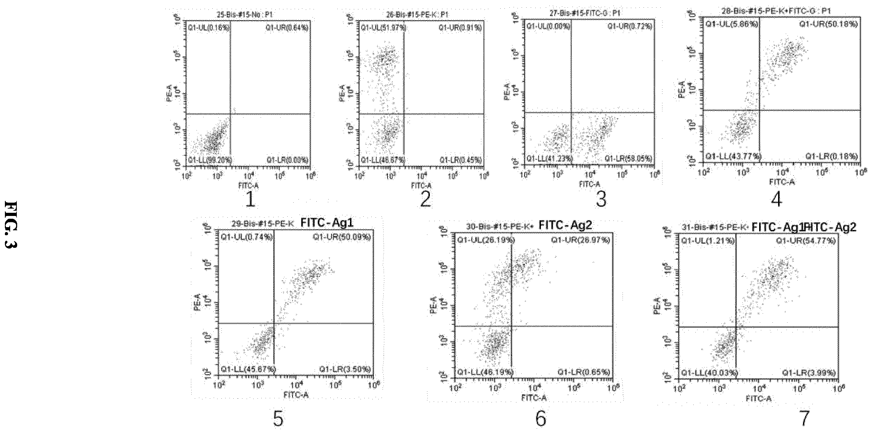

The results showed that, there were bispecific antigen-binding polypeptides expressed on the cell surface (FIG. 3). In FIG. 3:
1. No staining negative control, neither PE signal nor FITC signal;
2. PEK single-staining, more than 50% of the cells had PE signal, indicating the expression of Kappa light chain;
3. FITC-G single-staining, more than 50% of the cells had FITC signal, indicating the expression of IgG heavy chain;
4. PEK+FITC-G double-staining, more than 50% of the cells had dual signals of PE and PFITC, indicating that both the Kappa light chain and the IgG heavy chain were expressed;
5. PEK+FITC-Ag1 double-staining, more than 50% of the cells had dual signals of PE and PFITC, indicating that the antibodies expressed on the cell surface could bind to the PD1 antigen;
6. PEK+FITC-Ag2 double-staining, more than 26% of the cells had dual signals of PE and PFITC, indicating that the antibodies expressed on the cell surface could bind to the PDL1 antigen;
7. PEK+FITC-Ag1+FITC-Ag2 triple-staining, more than 54% of the cells had dual signals of PE and PFITC. Although the ratio of double-positive cells has only increased by 4%, the double-positive cell population shifted to the right, indicating that the FITC fluorescence signal was enhanced, which should be the result of the superposition of fluorescent signals caused by the binding of the antibodies displayed on the cell surface to both PD1 and PDL1 antigens.

### Example 3 Binding of the bispecific antigen-binding polypeptide to the antigen in a dose-dependent manner

According to the method of Example 2, the cells expressing the bispecific antigen-binding polypeptides were co-incubated with different concentrations of FITC-labeled PD1 antigens (FAg1, 0.3 µl, 1 µl, 3.3 µl) and different concentrations of FITC-labeled PDL1 antigens (FAg2, 0.3 µl, 1 µl, 3.3 µl), and analyzed by flow cytometry.

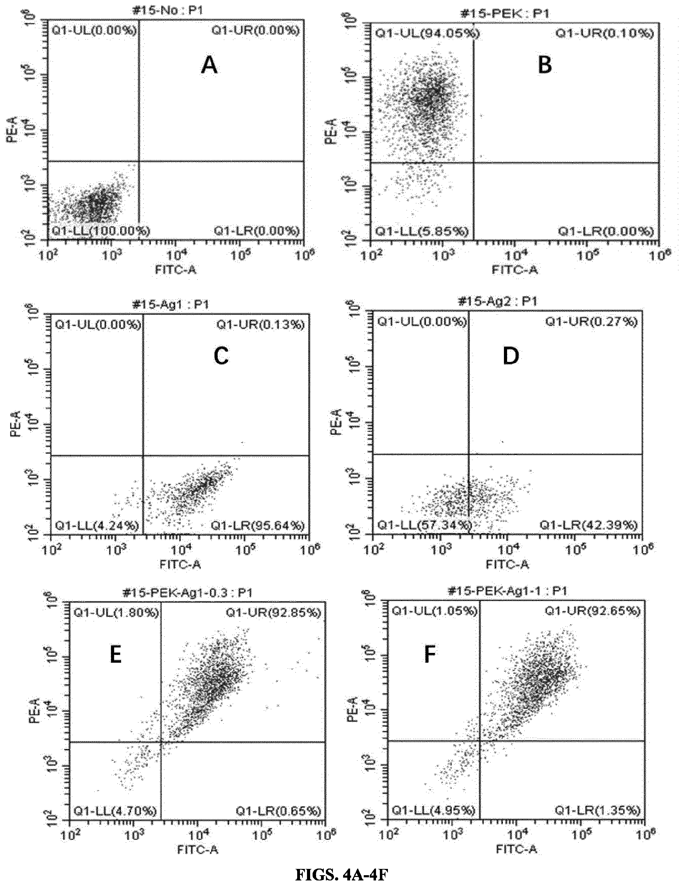

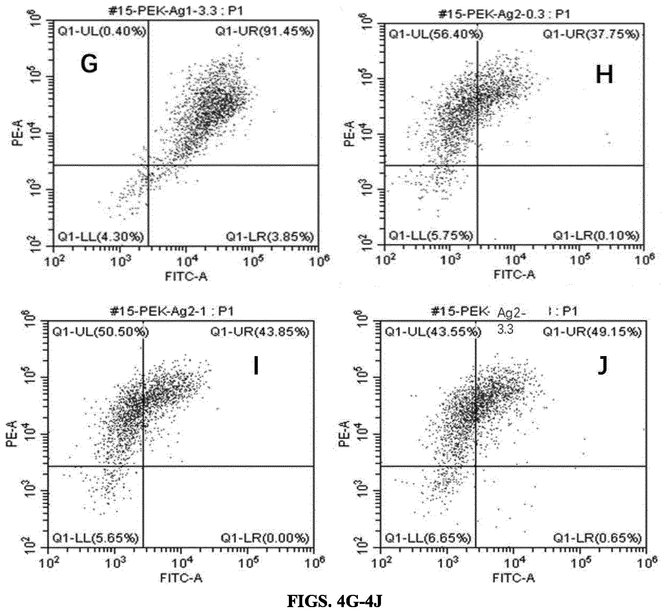

The results showed that, as the antigen concentration increased, the cell population shifted to the right, and the fluorescence signal increased with the increase of FAg, indicating that the bispecific antibodies showed dose-dependency to both antigens (FIG. 4). In FIG. 4: A. No staining negative control; B. PEK single-staining; C. FAgI single-staining; D. FAg2 single-staining; E. PEK+0.3 µl FAg1; F. PEK+1 µl FAg1; G. PEK+3.3 µl FAg1; H. PEK+0.3 µl FAg2; I. PEK+1 µl FAg2; J. PEK+3.3 µl Ag2.

## Claims

1. A method for constructing a bispecific antigen-binding polypeptide expression vector, comprising:
a) providing a first polynucleotide comprising S5-LC1-S6 in the direction from 5' to 3';
b) providing a second polynucleotide comprising B4-VH1-B3 in the direction from 5' to 3';
c) providing a third polynucleotide comprising B2-LC2-B4 in the direction from 5' to 3';
d) providing a fourth polynucleotide comprising B5-VH2-B6 in the direction from 5' to 3';
e) providing a fifth polynucleotide comprising S6-expression vector fragment I-B2 in the direction from 5' to 3';
f) providing a sixth polynucleotide comprising B3-expression vector fragment II-B5 in the direction from 5' to 3';
g) providing a seventh polynucleotide comprising B6-expression vector fragment III-S5 in the direction from 5' to 3';
h) specifically cleaving the first polynucleotide, the second polynucleotide, the third polynucleotide, the fourth polynucleotide, the fifth polynucleotide, the sixth polynucleotide and the seventh polynucleotide with a restriction endonuclease to obtain a cleaved first polynucleotide, a cleaved second polynucleotide, a cleaved third polynucleotide, a cleaved fourth polynucleotide, a cleaved fifth polynucleotide, a cleaved sixth polynucleotide and a cleaved seventh polynucleotide; wherein the restriction endonuclease specifically recognizes S5, S6, B4, B3, B2, B5 and B6, respectively;
i) mixing the cleaved first polynucleotide, the cleaved second polynucleotide, the cleaved third polynucleotide, the cleaved fourth polynucleotide, the cleaved fifth polynucleotide, the cleaved sixth polynucleotide and the cleaved seventh polynucleotide so that they can be ligated directionally and cyclized to form the expression vector;
wherein the LC1 encodes a first light chain of the bispecific antigen-binding polypeptide, the VH1 encodes a first heavy chain variable region of the bispecific antigen-binding polypeptide, and the first light chain can bind to the first heavy chain variable region to form a first Fab for recognizing a first target;
the LC2 encodes a second light chain of the bispecific antigen-binding polypeptide, the VH2 encodes a second heavy chain variable region of the bispecific antigen-binding polypeptide, and the second light chain can bind to the second heavy chain variable region to form a second Fab for recognizing a second target;
wherein the B2, B3, B4, B5, B6, S5 and S6 are each independently recognition sites for the restriction endonuclease.

2. The method according to claim 1, wherein the end produced from the specific cleavage of B2 by the restriction endonuclease that specifically recognizes it does not recognize or link to each other with the end produced from the specific cleavage of any one of the B3, B4, B5, B6, S5 and S6 by the corresponding restriction endonuclease.

3. The method according to any one of claims 1-2, wherein the end produced from the specific cleavage of B3 by the restriction endonuclease that specifically recognizes it does not recognize or link to each other with the end produced from the specific cleavage of any one of the B2, B4, B5, B6, S5 and S6 by the corresponding restriction endonuclease.

4. The method according to any one of claims 1-3, wherein the end produced from the specific cleavage of B4 by the restriction endonuclease that specifically recognizes it does not recognize or link to each other with the end produced from the specific cleavage of any one of the B2, B3, B5, B6, S5 and S6 by the corresponding restriction endonuclease.

5. The method according to any one of claims 1-4, wherein the end produced from the specific cleavage of B5 by the restriction endonuclease that specifically recognizes it does not recognize or link to each other with the end produced from the specific cleavage of any one of the B2, B4, B3, B6, S5 and S6 by the corresponding restriction endonuclease.

6. The method according to any one of claims 1-5, wherein the end produced from the specific cleavage of B6 by the restriction endonuclease that specifically recognizes it does not recognize or link to each other with the end produced from the specific cleavage of any one of the B2, B4, B5, B3, S5 and S6 by the corresponding restriction endonuclease.

7. The method according to any one of claims 1-6, wherein the end produced from the specific cleavage of S5 by the restriction endonuclease that specifically recognizes it does not recognize or link to each other with the end produced from the specific cleavage of any one of the B2, B4, B5, B6, B3 and S6 by the corresponding restriction endonuclease.

8. The method according to any one of claims 1-7, wherein the end produced from the specific cleavage of S6 by the restriction endonuclease that specifically recognizes it does not recognize or link to each other with the end produced from the specific cleavage of any one of the B2, B4, B5, B6, S5 and B3 by the corresponding restriction endonuclease.

9. The method according to any one of claims 1-8, wherein the restriction endonuclease is selected from SfiI and BsmBI.

10. The method according to any one of claims 1-9, wherein the B2, B3, B4, B5 and B6 can be specifically recognized and cleaved by BsmBI.

11. The method according to any one of claims 1-10, wherein the S5 and S6 can be specifically recognized and cleaved by Sfil.

12. The method according to any one of claims 1-11, wherein the B2 comprises a nucleic acid sequence as set forth in SEQ ID NO: 1.

13. The method according to any one of claims 1-12, wherein the B3 comprises a nucleic acid sequence as set forth in SEQ ID NO: 2.

14. The method according to any one of claims 1-13, wherein the B4 comprises a nucleic acid sequence as set forth in SEQ ID NO: 3.

15. The method according to any one of claims 1-14, wherein the B5 comprises a nucleic acid sequence as set forth in SEQ ID NO: 4.

16. The method according to any one of claims 1-15, wherein the B6 comprises a nucleic acid sequence as set forth in SEQ ID NO: 5.

17. The method according to any one of claims 1-16, wherein the S5 comprises a nucleic acid sequence as set forth in SEQ ID NO: 6.

18. The method according to any one of claims 1-17, wherein the S6 comprises a nucleic acid sequence as set forth in SEQ ID NO: 7.

19. The method according to any one of claims 1-18, further comprising introducing the first polynucleotide into a first bacterium to obtain an LC1 light chain component bacterial library.

20. The method according to claim 19, comprising inserting the first polynucleotide into a component vector to form an LC1 storage ligation product, and introducing the LC1 storage ligation product into the first bacterium to obtain the LC1 light chain component bacterial library.

21. The method according to claim 20, further comprising acquiring a first light chain component plasmid comprising the first polynucleotide from the LC1 light chain component bacterial library, and acquiring the cleaved first polynucleotide from the first light chain component plasmid.

22. The method according to claim 21, comprising digesting the first light chain component plasmid with a restriction endonuclease that specifically recognizes the S5 and S6, thus obtaining the cleaved first polynucleotide.

23. The method according to any one of claims 1-22, further comprising introducing the second polynucleotide into a second bacterium to obtain a VH1 heavy chain component bacterial library.

24. The method according to claim 23, comprising inserting the second polynucleotide into a component vector to form a VH1 storage ligation product, and introducing the VH1 storage ligation product into the second bacterium to obtain the VH1 heavy chain component bacterial library.

25. The method according to claim 24, further comprising acquiring a first heavy chain component plasmid comprising the second polynucleotide from the VH1 heavy chain component bacterial library, and acquiring the cleaved second polynucleotide from the first heavy chain component plasmid.

26. The method according to claim 25, comprising digesting the first heavy chain component plasmid with a restriction endonuclease that specifically recognizes the B4 and B3, thus obtaining the cleaved second polynucleotide.

27. The method according to any one of claims 1-26, further comprising introducing the third polynucleotide into a third bacterium to obtain an LC2 light chain component bacterial library.

28. The method according to claim 27, comprising inserting the third polynucleotide into a component vector to form an LC2 storage ligation product, and introducing the LC2 storage ligation product into the third bacterium to obtain the LC2 light chain component bacterial library.

29. The method according to claim 28, further comprising acquiring a second light chain component plasmid comprising the third polynucleotide from the LC2 light chain component bacterial library, and acquiring the cleaved third polynucleotide from the second light chain component plasmid.

30. The method according to claim 29, comprising digesting the second light chain component plasmid with a restriction endonuclease that specifically recognizes the B2 and B4, thus obtaining the cleaved third polynucleotide.

31. The method according to any one of claims 1-30, further comprising introducing the fourth polynucleotide into a fourth bacterium to obtain a VH2 heavy chain component bacterial library.

32. The method according to claim 31, comprising inserting the fourth polynucleotide into a component vector to form a VH2 storage ligation product, and introducing the VH2 storage ligation product into the fourth bacterium to obtain the VH2 heavy chain component bacterial library.

33. The method according to claim 32, further comprising acquiring a second heavy chain component plasmid comprising the fourth polynucleotide from the VH2 heavy chain component bacterial library, and acquiring the cleaved fourth polynucleotide from the second heavy chain component plasmid.

34. The method according to claim 33, comprising digesting the second heavy chain component plasmid with a restriction endonuclease that specifically recognizes the B5 and B6, thus obtaining the cleaved fourth polynucleotide.

35. The method according to any one of claims 1-34, further comprising introducing the fifth polynucleotide into a fifth bacterium to obtain an expression vector component I bacterial library.

36. The method according to claim 35, comprising inserting the fifth polynucleotide into a component vector to form an expression vector fragment I storage ligation product, and introducing the storage ligation product into the fifth bacterium to obtain the expression vector component I bacterial library.

37. The method according to claim 36, further comprising acquiring a display fragment component plasmid I comprising the fifth polynucleotide from the expression vector component I bacterial library, and acquiring the cleaved fifth polynucleotide from the display fragment component plasmid I.

38. The method according to claim 37, comprising digesting the display fragment component plasmid I with a restriction endonuclease that specifically recognizes the S6 and B2, thus obtaining the cleaved fifth polynucleotide.

39. The method according to any one of claims 1-38, further comprising introducing the sixth polynucleotide into a sixth bacterium to obtain an expression vector component II bacterial library.

40. The method according to claim 39, comprising inserting the sixth polynucleotide into a component vector to form an expression vector fragment II storage ligation product, and introducing the storage ligation product into the sixth bacterium to obtain the expression vector component II bacterial library.

41. The method according to claim 40, further comprising acquiring a display fragment component plasmid II comprising the sixth polynucleotide from the expression vector component II bacterial library, and acquiring the cleaved sixth polynucleotide from the display fragment component plasmid II.

42. The method according to claim 41, comprising digesting the display fragment component plasmid II with a restriction endonuclease that specifically recognizes the B3 and B5, thus obtaining the cleaved sixth polynucleotide.

43. The method according to any one of claims 1-42, further comprising introducing the seventh polynucleotide into a seventh bacterium to obtain an expression vector component III bacterial library.

44. The method according to claim 43, comprising inserting the seventh polynucleotide into a component vector to form an expression vector fragment III storage ligation product, and introducing the storage ligation product into the seventh bacterium to obtain the expression vector component III bacterial library.

45. The method according to claim 44, further comprising acquiring a display fragment component plasmid III comprising the seventh polynucleotide from the expression vector component III bacterial library, and acquiring the cleaved seventh polynucleotide from the display fragment component plasmid III.

46. The method according to claim 45, comprising digesting the display fragment component plasmid III with a restriction endonuclease that specifically recognizes the B6 and S5, thus obtaining the cleaved seventh polynucleotide.

47. The method according to claim 43-46, comprising cryopreserving the LC1 light chain component bacterial library, the LC2 light chain component bacterial library, the VH1 heavy chain component bacterial library, the VH2 heavy chain component bacterial library, the expression vector component I bacterial library, the expression vector component II bacterial library and/or the expression vector component III bacterial library.

48. The method according to any one of claim 20-47, wherein the component vector is derived from a pUC vector.

49. The method according to claim 48, wherein the pUC vector is a pUC19 vector or derived from a pUC19 vector.

50. The method according to any one of claims 19-49, wherein the LC1 light chain component bacterial library comprises at least 10 different clones.

51. The method according to any one of claims 23-50, wherein the VH1 heavy chain component bacterial library comprises at least 10 different clones.

52. The method according to any one of claims 27-51, wherein the LC2 light chain component bacterial library comprises at least 10 different clones.

53. The method according to any one of claims 31-52, wherein the VH2 heavy chain component bacterial library comprises at least 10 different clones.

54. The method according to any one of claims 1-53, wherein the bispecific antigen-binding polypeptide expression vector comprises at least 10 different clones.

55. The method according to any one of claims 1-54, wherein the first polynucleotide, the second polynucleotide, the third polynucleotide and/or the fourth polynucleotide are obtained from sample materials.

56. The method according to claim 55, wherein the sample materials comprise antibodies targeting specific antigens or antigen-binding fragments thereof.

57. The method according to claim 56, wherein the antibodies or antigen-binding fragments thereof target PD-1 and/or PD-L1.

58. The method according to any one of claims 1-57, wherein the directional ligation involves using a ligase.

59. The method according to claim 58, wherein the ligase comprises T4 DNA ligase.

60. A bispecific antigen-binding polypeptide expression vector produced by the method according to any one of claims 1-59.

61. A bispecific antigen-binding polypeptide display library established by utilizing the bispecific antigen-binding polypeptide expression vector of claim 60.

62. The library according to claim 61, which is a mammalian cell display library.

63. The library according to any one of claims 60-61, which is capable of displaying at least 10 different bispecific antibodies or antibody fragments thereof.

64. A method for screening antibodies or antibody fragments, comprising utilizing the library according to any one of claims 61-63.
